(19) Europäisches Patentamt
European Patent Office
Office européen des brevets

(11) Publication number: **0 491 326 A2**

# (12) EUROPEAN PATENT APPLICATION

(21) Application number: **91121545.7**

(22) Date of filing: **16.12.91**

(51) Int. Cl.$^5$: **C07C 51/15**, C07C 67/343, C07C 29/40

(30) Priority: **17.12.90 IT 2240390**

(43) Date of publication of application: **24.06.92 Bulletin 92/26**

(84) Designated Contracting States: **AT BE CH DE DK ES FR GB GR IT LI LU NL SE**

(71) Applicant: **ISTITUTO GUIDO DONEGANI S.p.A.**
**4, Via Fauser**
**I-28100 Novara(IT)**

(72) Inventor: **Castaldi, Graziano, Dr.**
**Via Livia Gallina**
**I-28072 Briona, Novara(IT)**
Inventor: **Borsotti, Giampiero**
**14, Strada Pastore**
**I-28100 Novara(IT)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

## (54) Process for the functionalization of trifluoromethylbenzenes.

(57) Substituted trifluoromethylbenzenes are selectively functionalized by means of an initial metalation of the particular substrate with a metalating agent chosen from mixtures of lithium-and magnesium-alkyls and mixtures of lithium alkyls and magnesium salts and subsequent reaction of the product thus obtained with an electrophile.

EP 0 491 326 A2

The present invention relates to a process for the selective functionalization of trifluoromethylbenzenes, which comprises the reaction of a specific substrate with a suitable metalating agent and the subsequent reaction of the product thus obtained with an electrophile.

More specifically, the present invention relates to a process for the preparation of substituted trifluoromethylbenzenes, in which the desired substituent enters into a strictly predetermined position and the desired substituted product is formed with high yields and selectivity, in spite of the fact that said substitution competes with other potential substitution reactions.

It is known that 1,3-bis-trifluoromethylbenzene can be transformed into the corresponding benzoic acid by means of initial metalation with lithium alkyls and subsequent reaction with $CO_2$. Similarly, from the article in Journal of Organometallic Chemistry (1974), vol. 57, page 321, it can be taken that it is possible to effect the following reaction:

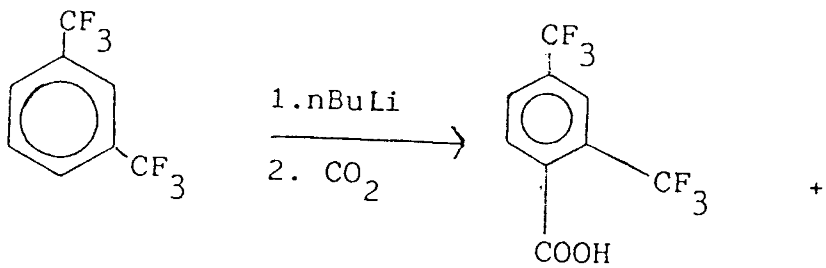


(1)

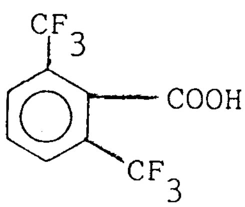

(2)

wherein the ratio of products (1) and (2) is 60/40.

In addition, the article in J. Chem. Soc. (1971), page 3305 describes the following reaction:

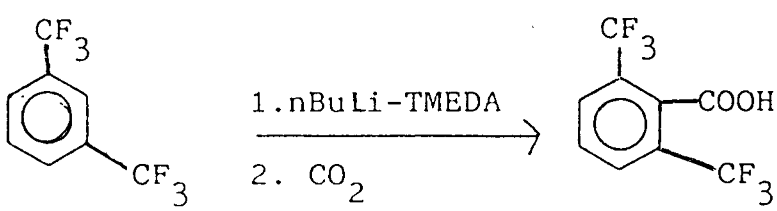


(2)

in which the presence of the complexing agent TMEDA (tetramethylethylenediamine) favours the exclusive formation of compound (2).

On the other hand, the importance of compound (1) or 2,4-bis-trifluoromethylbenzene derivatives in the preparation of compounds useful in various fields is also well-known.

For example, reference can be made to herbicides of formula

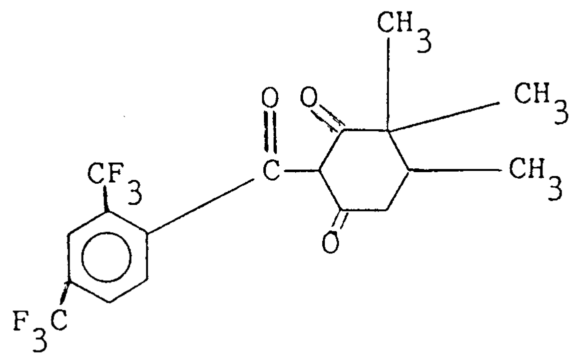

described in EP-A-186 120, or of formula

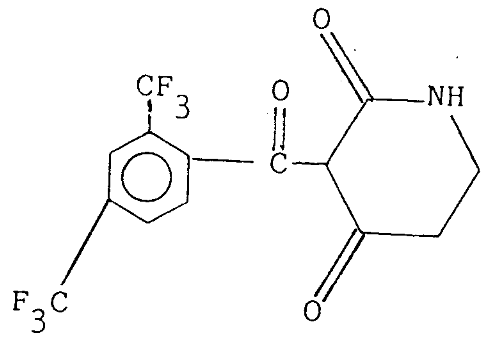

described in EP-A-278 742; or products used in the pharmaceutical field, for example, in the treatment of malaria, such as that of formula

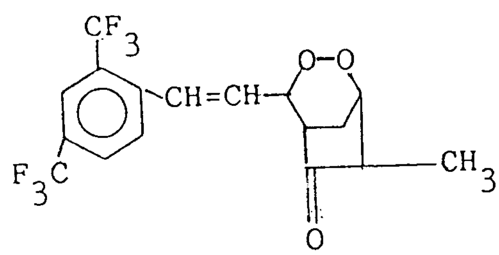

as described in EP-A-311 955, or in the prevention of coronary disorders, such as that of formula:

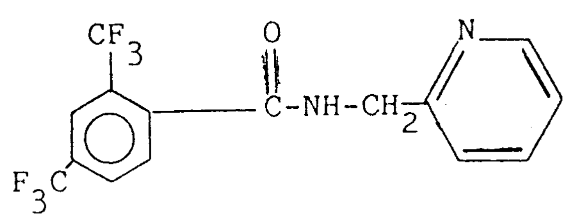

as described in US-A-3,946,027.

Other interesting uses of substituted aromatic products belonging to type (1) are found in the dye industry (J. Soc. Dyers Colour. 1982, 98 (1), 10-13), photographic field (JP-A-59/69755), or in the preparation of suitable compounds for the production of apparatus in the non-linear optical industry (JP-A-58/150523).

All previous references, which probably represent only a small part of those existing, make it clear how important it is to have readily available an intermediate of type (1), whereas in fact, as far as is known at the moment, either it cannot be obtained or it can be obtained only in admixture with an undesired product (of

no use) from which it must be separated by purification which is not always easy to carry out and which in any case does not favourably influence the economy of an industrial process intended to prepare useful products including, for example, those listed above.

It has now been found that industrial quantities of compound (1) and more generally substituted trifluoromethylbenzenes, can be prepared by means of an initial reaction of the specific substrate with certain metalating agents and subsequent reaction of the metalated product thus obtained with the desired electrophilic agent.

The functionalization of the trifluoromethylbenzene substrate takes place selectively in the desired position, bearing in mind the intended purpose of the substituted product: if there is only one $-CF_3$ group present, the substitution will always take place in an ortho position with respect to this group; if other substituents are present, the positioning of the new substituent will naturally be determined by the kind of substituents already present and their position with respect to the $-CF_3$ group. It is obviously possible to choose trifluoromethylbenzenes containing suitable activating groups to direct the insertion of functional groups in accordance with the intended final purpose.

Accordingly, the present invention provides a process for the functionalization of aromatic substrates of general formula (I)

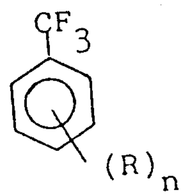

(I)

in which n is zero or an integer of from 1 to 4, and R is an activating group (when n>1, the groups R may be the same or different) selected from $-CF_3$, amino, mono- and dialkylamino, aminoalkyl, hydroxy, hydroxyalkyl, ether (e.g. alkoxy), ketal, acetal and other groups. In said groups the alkyl moieties thereof usually have from 1 to 10, preferably 1 to 6 and particularly 1 to 4 carbon atoms, such as ethyl, methyl and propyl.

As already mentioned, the functionalization process according to the present invention comprises the reaction of the specific substrate, selected from those listed above, with a metalating agent selected from mixtures of lithium and magnesium alkyls and mixtures of lithium alkyls and magnesium salts. Preferred lithium and magnesium alkyls are those having 1 to 6, particularly 1 to 4 carbon atoms in the alkyl portion thereof.

The reaction is usually carried out in solution at temperatures ranging from -80 °C to +80 °C, the range from -20 °C to +20 °C being preferred.

Examples of suitable solvents are THF, ethyl ether, benzene, toluene, pentane, hexane, heptane and cyclohexane.

Particularly effective metalating agents are mixtures of n-butyllithium and dibutyl magnesium, or a mixture of n-butyllithium and magnesium bromide. (Other magnesium salts which can be used are, e.g., the chloride, iodide, fluoride, sulfate, nitrate, etc.)

Preferred examples of suitable substrates to be subjected to the metalation reaction are: 1,3-bis-trifluoromethylbenzene; 1-methoxy-3-trifluoromethylbenzene and N,N-dimethylamino-3-trifluoromethylbenzene.

The metalated product obtained from the previous treatment is caused to react with an electrophilic agent to obtain the desired functionalized compound.

Any organic compound capable of undergoing a nucleophilic substitution reaction can be employed as electrophilic agent.

Examples of suitable electrophilic reagents are $CO_2$, dialkylcarbonates, urea, formamides, amides and esters of carboxylic acids, halo- nd dihaloalkyls, halogens (e.g. chlorine, fluorine, bromine and iodine), metallic salts, sulfones, aldehydes, ketones, anhydrides and nitriles.

The reaction between the metalated substrate and the electrophilic agent can be carried out after separation of the former, or as a one-pot process directly in the apparatus in which the metalation reaction has taken place, preferably at a temperature ranging from -80 °C to +80 °C.

EXAMPLE 1

**Preparation of 2,4-bis-trifluoromethylbenzoic acid.**

A solution of dibutylmagnesium in heptane (0.9 M, 10 mmoles, 11 ml) is dropped, under vacuum and at room temperature, into a solution of 1,3-bis-trifluoromethyl benzene (2.14 g, 10 mmoles) in ethyl ether (10 ml).

The solution thus obtained is cooled to 0 °C and a solution of n-butyllithium in hexane (2.5 M, 10 mmoles, 4 ml) is added. The reaction mixture is kept at 20 °C for 4 hours, and is then poured onto solid carbon dioxide in fine powder form, and kept under vigorous stirring.

The mixture is then left to reach room temperature and acidified to pH 1-2 with 0.1 M HCl. The resulting reaction mixture is extracted with dichloromethane and the phases are separated.

The organic phase is extracted with 10% aqueous NaOH and the basic water extract is acidified with HCl to pH 1.2, whereafter said water extract is extracted with dichloromethane and the resulting organic extract is washed with water and dried over sodium sulphate.

Upon evaporation of the solvent under vacuum, 2,4-bis-trifluoromethylbenzoic acid (1.8 g), b.p. 107-109 °C, is obtained.

$^1H$-NMR ($CDCl_3$) $\delta$ (ppm): 7.95 (m, 1H), 8.06 (m, 1H), 8.12 (m, 1H), 11.8 (broad, 1H).

EXAMPLE 2

**Preparation of 2,4-trifluoromethylbenzyl alcohol**.

Using the same procedure as described in example 1 and using paraformaldehyde (1 g) instead of solid carbon dioxide, the title compound is obtained after chromatographic purification of the crude reaction product on silica gel. $^1H$-NMR ($CDCl_3$) $\delta$ (ppm): 7.86 - 7.70 (m, aromatic protons, 3H), 4.85 (s, 2H).

EXAMPLE 3

**Preparation of methyl-2,4-bis-trifluoromethylbenzoate**

Using the same procedure as described in example 1 and using dimethylcarbonate (2 g) instead of solid carbon dioxide, the desired product (2 g) is obtained, after chromatographic purification of the crude reaction product on silica gel. $^1H$-NMR ($CDCl_3$) $\delta$ (ppm): 8.2 - 7.6 (m, aromatic protons, 3H), 3.93 (s, 3H).

**Claims**

**1.** Process for the selective functionalization of trifluoromethylbenzenes, comprising the reaction of a compound of general formula (I):

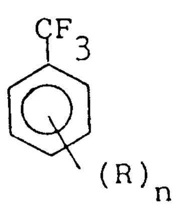

(I)

where n is zero or an integer of from 1 to 4, the groups R, the same or different from each other, are activating groups selected from $-CF_3$, amino, aminoalkyl, hydroxy, hydroxyalkyl, alkylamino, ether, ketal and acetal groups; with a metalating agent selected from mixtures of lithium and magnesium alkyls and mixtures of lithium alkyls and magnesium salts and the subsequent reaction of the resulting product with an electrophilic agent.

**2.** Process according to claim 1, in which the electrophilic agent is chosen from $CO_2$, dialkylcarbonates, urea, formamides, amides, esters, halo- and dihaloalkanes, halogens, metallic salts, sinfones, aldehydes, ketones, anhydrides, nitriles and mixtures thereof.

**3.** Process according to any one of claims 1 and 2, in which the metalation reaction is carried out at a temperature of from -80 °C to +80 °C.

4. Process according to any one of claims 1 to 3, in which the metalation reaction is carried out at a temperature of from -20 °C to +20 °C.

5. Process according to any one of claims 1 to 4, in which the metalation reaction is carried out in the presence of a solvent, preferably selected form tetrahydrofuran, diethyl ether, benzene, toluene, pentane, hexane, heptane, cyclohexane and mixtures thereof.

6. Process according to any one of claims 1 to 5, in which the metalating agent is selected from mixtures of butyllithium and dibutyl magnesium and mixtures of butyllithium and magnesium bromide.

7. Process according to any one of claims 1 to 6, in which the reaction with the electrophilic agent is carried out in the same environment and under the same conditions as the metalation reaction.